# EUROPEAN PATENT SPECIFICATION

(11) **EP 4 009 874 B1**
(45) Date of publication and mention of the grant of the patent: **07.10.2026**
(21) Application number: 20771764.6
(22) Date of filing: 05.08.2020
(51) Int. Cl.: A61B 8/00, G01S 7/52

(54) **ULTRASOUND SYSTEM WITH ACOUSTIC OUTPUT CONTROL USING IMAGE DATA**
AKUSTISCHE AUSGANGSSTEUERUNG EINES ULTRASCHALLSYSTEMS UNTER VERWENDUNG VON BILDDATEN
COMMANDE DE SORTIE ACOUSTIQUE DE SYSTÈME ULTRASONORE À L'AIDE DE DONNÉES D'IMAGE

(30) Priority: 05.08.2019 US 201962882660 P
(43) Date of publication of application: 15.06.2022
(73) Proprietor: Koninklijke Philips N.V., 5656 AE Eindhoven (NL)
(72) Inventor: OWEN, Neil Reid, 5656 AE Eindhoven (NL); LOFLIN, Chris, 5656 AE Eindhoven (NL); DONLON, John Gerard, 5656 AE Eindhoven (NL)
(74) Representative: Philips Intellectual Property & Standards
(86) International application number: PCT/EP2020/071943
(87) International publication number: WO 2021/023753

(56) References cited:
- US-A1- 2004 102 703
- US-A1- 2018 103 912

## Description

This invention relates to medical diagnostic ultrasound systems and, in particular, to the control of the acoustic output of an ultrasound probe using image data.

US 2004/102703 A1 discloses a method aimed at improving ultrasound image quality based on a mode in which high-power ultrasonic pulses are temporarily generated to provide for better signal-to-noise ratio.

US 2018/103912 A1 discloses an ultrasound system with a deep learning neural network for identifying anatomy in images, and for assessing image quality.

Ultrasonic imaging is one of the safest of the medical imaging modalities as it uses non-ionizing radiation in the form of propagated acoustic waves. Nevertheless, numerous studies have been conducted over the years to determine possible bioeffects. These studies have been focused on long-term exposure to ultrasonic energy which may have thermal effects, and cavitational effects due to high peak pulse energy. Among the more prominent studies and reports published on these effects are "Bioeffects and Safety of Diagnostic Ultrasound," AIUM Report, January 28, 1993, and "American Institute of Ultrasound in Medicine Bioeffects Consensus Report," Journal of Ultrasound in Medicine, vol. 27, issue 4, April 2008. The FDA also issues guidance documents on ultrasound safety and energy limits which are used in the FDA's clearance process, *e.g.*, "Information for Manufacturers Seeking Marketing Clearance of Diagnostic Ultrasound Systems and Transducers," FDA, September 2008. Manufacturers use all of this information and other sources when they are

designing, testing, and setting the energy limits for their ultrasound systems and transducer probes.

The measurement of the acoustic output from transducer probes is an integral part of the transducer design process. Measurements of acoustic output of probes under development can be made in a water tank and used to set the limits for driving the

probe transmitters in the ultrasound system. Currently, manufacturers are adhering to acoustic limits for general imaging of I_{spta.3}≤720mW/cm2 for thermal effect limitation and MI≤1.9 as the peak mechanical index for peak pulse (cavitation) effect limitation. The current operating levels for these thermal and mechanical measures are constantly displayed on the display screen with the image during operation of an ultrasound probe.

However, while an ultrasound system has these designed-in bioeffect limits, it is the responsibility of the clinician conducting an exam to see that the system is always operated safely, particularly for exams for which lower limits are recommended. An important consideration is that bioeffects are a function of not just output power, but other operating parameters such as imaging mode, pulse repetition frequency, focus depth, pulse length, and transducer type, which can also affect patient safety. There are some types of exams for which operational guidance recommends against certain probe operation. For example, shear wave imaging is contraindicated for obstetrical exams. Most ultrasound systems have some form of an acoustic output controller, which is constantly assessing these parameters and continually estimating the acoustic output and making adjustments to maintain operation within prescribed safety limits. However, more could be done beyond just measuring ultrasound system operating parameters. It would be desirable to automatically assess the conduct of an exam from the clinician's perspective and make output control adjustments or recommendations. It would be desirable, for instance, to characterize the anatomy being imaged and use image information in setting or recommending changes to acoustic output for improved patient safety.

The invention is defined by the independent claims. The dependent claims define advantageous embodiments.

In accordance with the principles of the present invention, an ultrasound system uses image recognition to characterize the anatomy being imaged, then considers an identified anatomical characteristic to set the level or limit of acoustic output of an ultrasound probe. Alternatively, instead of automatically setting the acoustic output level or limit, the system can alert the clinician that a change in operating levels or conditions would be prudent for the present exam. In these ways, the clinician is able to maximize the signal-to-noise level in the images for clearer, more diagnostic images while maintaining a safe level of acoustic output for patient safety.

In the drawings:
FIGURE 1 illustrates the steps of a method for using acquired image data to advise or change acoustic output in accordance with the present invention.
FIGURE 2 is a block diagram of an ultrasound system constructed in accordance with a first implementation of the present invention which uses an anatomical model to identify the anatomy in an ultrasound image.
FIGURE 3 illustrates the steps of a method for operating the ultrasound system of FIGURE 2 in accordance with the principles of the present invention.
FIGURE 4 is a block diagram of an ultrasound system constructed in accordance with a second implementation of the present invention which uses a neural network model to identify the anatomy in an ultrasound image in accordance with the present invention.

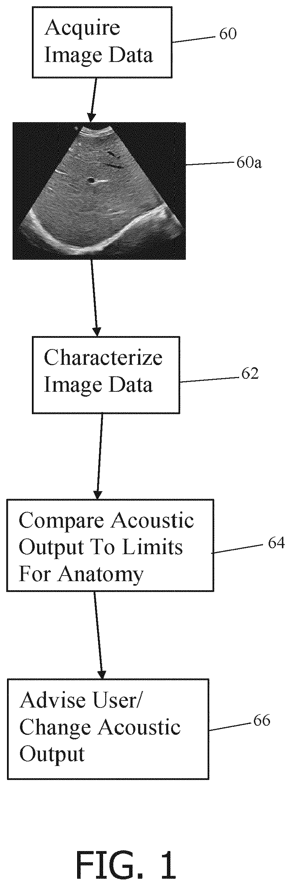

Referring first to FIGURE 1, a method for using image data in the control of acoustic output is shown. Image data is acquired in step 60 as a clinician scans a patient. In the example of FIGURE 1, the clinician is scanning the liver as shown by acquired liver image 60a. The ultrasound system identifies this image as a liver image by recognizing known characteristics of a liver image, such as its depth in the body, the generally smooth texture of the liver tissue, the depth to its far boundary, the presence of bile ducts and blood vessels, and the like. The ultrasound system can also consider cues from the exam setup such as the use of a deep abdominal probe and the extensive depth of the image. The ultrasound system uses this information to characterize the image data in step 62 as being an image of the liver acquired in an abdominal imaging exam. The ultrasound system then identifies the current acoustic output of the probe using probe operating characteristics such as the drive voltage, the thermal and MI settings, and the other probe setup parameters listed above. The calculated acoustic output is then compared with the recommended clinical limits for an abdominal exam at step 64.

An advisory or adjustment step 66 determines whether additional action is indicated based on the comparison step 64. For example, if the present acoustic output is below the acoustic output limits recommended for the anatomy being imaged, a message can be issued to the clinician, advising that the acoustic output can be increased to generate echoes with stronger signal to noise levels and hence produce a clearer, sharper image. Other comparisons may indicate that the acoustic output is higher than recommended limits for the anatomy being imaged, or that a mode of operation is inappropriate for the anatomy being imaged.

The system then, if necessary, issues a message at step 66 that advises the clinician to adjust the acoustic output. The system may also responsively and automatically adjust the acoustic output limits to those recommended for an abdominal exam. If the present acoustic output is below the acoustic output limits recommended for the anatomy being imaged, a message can be issued to the clinician, advising that the acoustic output can be increased to generate echoes with stronger signal to noise levels and hence produce a clearer, sharper image.

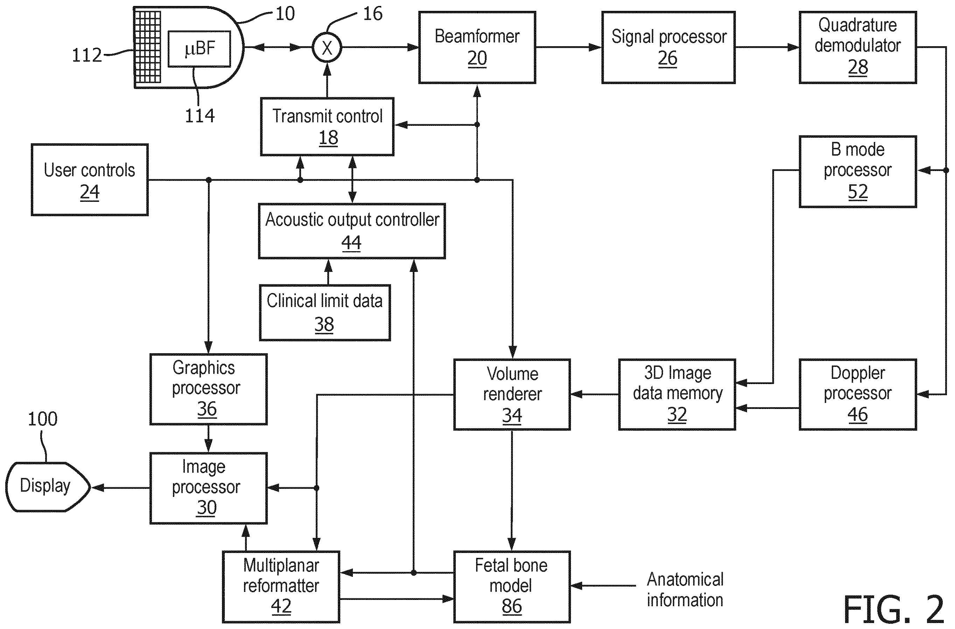

FIGURE 2 illustrates a first implementation of an ultrasound system in block diagram form, which system is capable of operating in accordance with the method of FIGURE 1. A transducer array 112 is provided in an ultrasound probe 10 for transmitting ultrasonic waves and receiving echo information from a region of the body. The transducer array 112 may be a two-dimensional array of transducer elements capable of electronically scanning in two or three dimensions, in both elevation (in 3D) and azimuth, as shown in the drawing. Alternatively, the transducer may be a one-dimensional array capable of scanning a single image plane. The transducer array 112 is coupled to a microbeamformer 114 in the probe which controls transmission and reception of signals by the array elements. Microbeamformers are capable of at least partial beamforming of the signals received by groups or "patches" of transducer elements as described in US Pats. 5,997,479 (Savord et al.), 6,013,032 (Savord), and 6,623,432 (Powers et al.) A one-dimensional array transducer can be operated directly by a system beamformer without the need for a microbeamformer. The microbeamformer in the probe implementation shown in FIGURE 2 is coupled by a probe cable to a transmit/receive (T/R) switch 16 which switches between transmission and reception and protects the main system beamformer 20 from high energy transmit signals. The transmission of ultrasonic beams from the transducer array 112 under control of the microbeamformer 114 is directed by a transmit controller 18 coupled to the T/R switch and the beamformer 20, which receives input from the user's operation of the system's user interface or controls 24. Among the transmit characteristics controlled by the transmit controller are the spacing, amplitude, phase, frequency, repetition rate, and polarity of transmit waveforms. Beams formed in the direction of pulse transmission may be steered straight ahead from the transducer array, or at different angles for a wider sector field of view.

The echoes received by a contiguous group of transducer elements are beamformed by appropriately delaying them and then combining them. The partially beamformed signals produced by the microbeamformer 114 from each patch are coupled to a main beamformer 20 where partially beamformed signals from individual patches of transducer elements are delayed and combined into a fully beamformed coherent echo signal. For example, the main beamformer 20 may have 128 channels, each of which receives a partially beamformed signal from a patch of 12 transducer elements. In this way the signals received by over 1500 transducer elements of a two-dimensional array transducer can contribute efficiently to a single beamformed signal. When the main beamformer is receiving signals from elements of a transducer array without a microbeamformer, the number of beamformer channels is usually equal to or more than the number of elements providing signals for beam formation, and all of the beamforming is done by the beamformer 20.

The coherent echo signals undergo signal processing by a signal processor 26, which includes filtering by a digital filter and noise reduction as by spatial or frequency compounding. The digital filter of the signal processor 26 can be a filter of the type disclosed in U.S. Patent No. 5,833,613 (Averkiou et al.), for example. The processed echo signals are demodulated into quadrature (I and Q) components by a quadrature demodulator 28, which provides signal phase information and can also shift the signal information to a baseband range of frequencies.

The beamformed and processed coherent echo signals are coupled to a B mode processor 52 which produces a B mode image of structure in the body such as tissue. The B mode processor performs amplitude (envelope) detection of quadrature demodulated I and Q signal components by calculating the echo signal amplitude in the form of (I²+Q²)½. The quadrature echo signal components are also coupled to a Doppler processor 46, which stores ensembles of echo signals from discrete points in an image field which are then used to estimate the Doppler shift at points in the image with a fast Fourier transform (FFT) processor. The Doppler shift is proportional to motion at points in the image field, *e.g.,* blood flow and tissue motion. For a color Doppler image, which may be formed for analysis of blood flow, the estimated Doppler flow values at each point in a blood vessel are wall filtered and converted to color values using a look-up table. Either the B mode image or the Doppler image may be displayed alone, or the two shown together in anatomical registration in which the color Doppler overlay shows the blood flow in tissue and vessels in the imaged region.

The B mode image signals and the Doppler flow values in the case of volume imaging are coupled to a 3D image data memory 32, which stores the image data in x, y, and z addressable memory locations corresponding to spatial locations in a scanned volumetric region of a subject. For 2D imaging a two dimensional memory having addressable x,y memory locations may the used. The volumetric image data of the 3D data memory is coupled to a volume renderer 34 which converts the echo signals of a 3D data set into a projected 3D image as viewed from a given reference point as described in US Pat. 6,530,885 (Entrekin et al.) The reference point, the perspective from which the imaged volume is viewed, may be changed by a control on the user interface 24, which enables the volume to be tilted or rotated to diagnose the region from different viewpoints. The rendered 3D image is coupled to an image processor 30, which processes the image data as necessary for display on an image display 100. The ultrasound image is generally shown in conjunction with graphical data produced by a graphics processor 36, such as the patient's name, image depth markers, and scanning information such as the probe thermal output and mechanical index MI. The volumetric image data is also coupled to a multiplanar reformatter 42, which can extract a single plane of image data from a volumetric dataset for display of a single image plane.

In accordance with the principles of the present invention, the system of FIGURE 2 has an image recognition processor. In the example of the implementation of FIGURE 2, the image recognition processor is a fetal bone model 86. The fetal model comprises a memory which stores a library of differently sized and/or shaped mathematical models in data form of typical fetal bone structures, and a processor which compares the models with structure in acquired ultrasound images. The library may contain different sets of models, each representing typical fetal structure at a particular age of fetal development, such as the first and second trimesters of development, for instance. The models are data representing meshes of bones of the fetal skeleton and skin (surface) of a developing fetus. The meshes of the bones are interconnected as are the actual bones of a skeleton so that their relative movements and ranges of articulation are constrained in the same manner as are those of an actual skeletal structure. Similarly, the surface mesh is constrained to be within a certain range of distance of the bones it surrounds. When the ultrasound image of an abdominal exam contains echoes which may be strong reflections from hard objects like bone, the image information is coupled to the fetal model and used to select a particular model from the library as the starting point for analysis. The models are deformable within constraint limits, *e.g.,* fetal age, by altering parameters of a model to warp the model, such as an adaptive mesh representing an approximate surface of a typical skull or femur, and thereby fit the model by deformation to structural landmarks in the image data set. An adaptive mesh model is desirable because it can be warped within the limits of its mesh continuity and other constraints in an effort to fit the deformed model to structure in an image. The foregoing model deformation and fitting is explained in further detail in international patent application number WO 2015/019299 (Mollus et al.) entitled "MODEL-BASED SEGMENTATION OF AN ANATOMICAL STRUCTURE." See also international patent application number WO 2010/150156 (Peters et al.) entitled "ESTABLISHING A CONTOUR OF A STRUCTURE BASED ON IMAGE INFORMATION" and US pat. appl. pub. no. 2017/0128045 (Roundhill et al.) entitled "TRANSLATION OF ULTRASOUND ARRAY RESPONSIVE TO ANATOMICAL ORIENTATION." This process is continued by an automated shape processor until data is found in a plane or volume which can be fitted by the model and thus identified as fetal bone structure. The planes in a volumetric image dataset may be selected by the fetal model operating on the volumetric image data provided by the volume renderer 34, when the bone model is configured to do this. Alternatively, a series of differently oriented image planes intersecting a suspected location can be extracted from the volume data by the multiplanar reformatter 42 and provided to the fetal model 86 for analysis and fitting. When the image analysis identifies fetal bone structure in an image, this characterization of the image data is coupled to an acoustic output controller 44, which compares the current acoustic output set by the controller with clinical limit data for an obstetrical exam, which is stored in a clinical limit data memory 38. If the current acoustic output setting is found to exceed a limit recommended for an obstetrical exam, the acoustic output controller can command the display of a message on the display 100, advising the clinician that a lower acoustic output setting is recommended. Alternatively, the acoustic output controller can set lower acoustic output limits for the transmit controller 18.

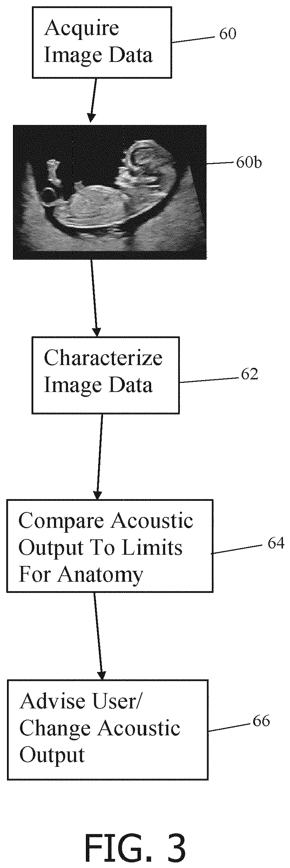

FIGURE 3 illustrates a method for controlling acoustic output using the ultrasound system of FIGURE 2 as immediately described above. Image data is acquired in step 60, which in this example is a fetal image 60b. The image data is analyzed by the fetal bone model which identifies fetal bone structure and thus characterizes the image as a fetal image in step 62. In step 64 the acoustic output controller 44 compares the current acoustic output performance and/or settings with the limits appropriate for a fetal exam. If any of those limits are exceeded by the present acoustic output, the user is advised to reduce acoustic output, or the acoustic output is automatically changed by the acoustic output controller in step 66. Alternatively, an imaging mode which is not recommended for obstetrical exams such as shear wave imaging can be automatically inhibited from operating.

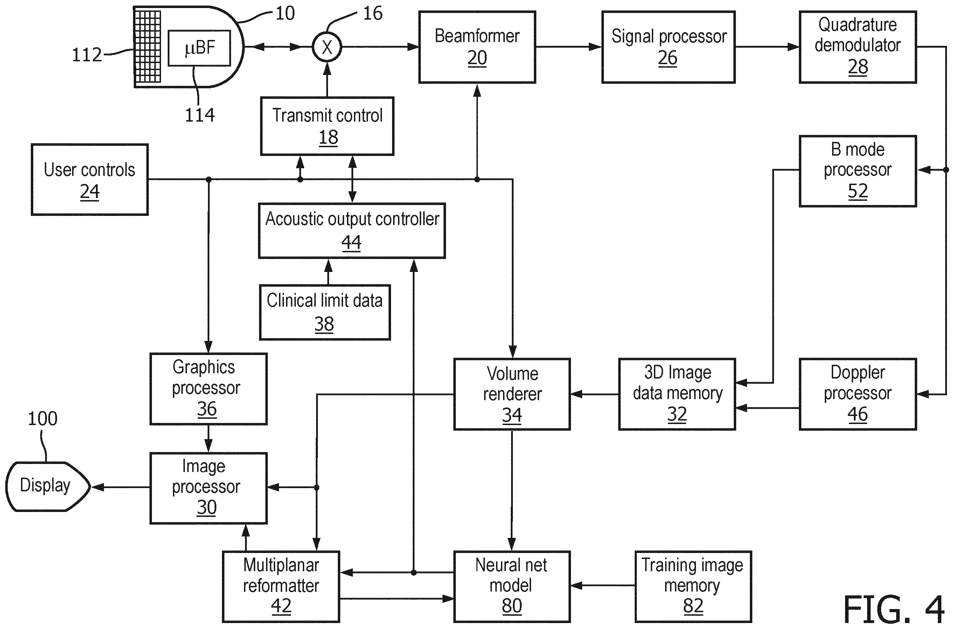

A second implementation of an ultrasound system of the present invention is illustrated in block diagram form in FIGURE 4. In the system of FIGURE 4, system elements which were shown and described in FIGURE 2 are used for like functions and operations and will not be described again. In the system of FIGURE 4 the image recognition processor comprises a neural network model 80. A neural network model makes use of a development in artificial intelligence known as "deep learning." Deep learning is a rapidly developing branch of machine learning algorithms that mimic the functioning of the human brain in analyzing problems. The human brain recalls what was learned from solving a similar problem in the past, and applies that knowledge to solve a new problem. Exploration is underway to ascertain possible uses of this technology in a number of areas such as pattern recognition, natural language processing and computer vision. Deep learning algorithms have a distinct advantage over traditional forms of computer programming algorithms in that they can be generalized and trained to recognize image features by analyzing image samples rather than writing custom computer code. The anatomy visualized in an ultrasound system would not seem to readily lend itself to automated image recognition, however. Every person is different, and anatomical shapes, sizes, positions and functionality vary from person to person. Furthermore, the quality and clarity of ultrasound images will vary even when using the same ultrasound system. That is because body habitus will affect the ultrasound signals returned from the interior of the body which are used to form the images. Scanning a fetus through the abdomen of an expectant mother, for example, will often result in greatly attenuated ultrasound signals and poorly defined anatomy in the fetal images. Nevertheless, the system described in this implementation has demonstrated the ability to use deep learning technology to recognize anatomy in fetal ultrasound images through processing by a neural network model. The neural network model is first trained by presenting to it a plurality of images of known anatomy, such as fetal images with known fetal structure which is identified to the model. Once trained, live images acquired by a clinician during an ultrasound exam are analyzed by the neural network model in real time, which identifies the fetal anatomy in the images.

Deep learning neural net models comprise software which may be written by a software designer, and are also publicly available from a number of sources. In the ultrasound system of FIGURE 4, the neural network model software is stored in a digital memory. An application which can be used to build a neural net model called "NVidia Digits" is available at https://developer.nvidia.com/digits. NVidia Digits is a high-level user interface around a deep learning framework called "Caffe" which has been developed by the Berkley Vision and Learning Center, http://caffe.berkeleyvision.org/. A list of common deep learning frameworks suitable for use in an implementation of the present invention is found at https://developer.nvidia.com/deep-learning-frameworks. Coupled to the neural network model 80 is a training image memory 82, in which ultrasound images of known fetal anatomy including fetal bone structure are stored and used to train the neural net model to identify that anatomy in ultrasound image datasets. Once the neural network model is trained by a large number of known fetal images, the neural network model receives image data from the volume renderer 34. The neural net model may receive other cues in the form of anatomical information such as the fact that an abdominal exam is being performed, as described above. The neural network model then analyzes regions of an image until fetal bone structure is identified in the image data. As before, the ultrasound system then characterizes the acquired ultrasound image as a fetal image, and forwards this characterization to the acoustic output controller 44. The acoustic output controller compares the currently controlled acoustic output with recommended clinical limits for fetal imaging, and alerts the user to excessive acoustic output or automatically resets acoustic output limit settings as described above for the first implementation.

Variations of the systems and methods described above will readily occur to those skilled in the art. Other image recognition algorithms may be employed if desired. Other apparatus and techniques can also or alternatively be used to characterize the anatomy in an image, such as data entered into the ultrasound system by a clinician.

The techniques of the present invention can be used in other diagnostic areas besides abdominal imaging. For instance, numerous ultrasound exams require standard views of anatomy for diagnosis, which are susceptible to relatively easy identification in an image. In diagnoses of the kidney, a standard view is a coronal image plane of the kidney. In cardiology, two-chamber, three-chamber, and four-chamber views of the heart are standard views. Models of other anatomy such as heart models are presently commercially available. A neural network model can be trained to recognize such views and anatomy in image datasets of the heart and then used to characterize cardiac use of an ultrasound probe. Other applications will readily occur to those skilled in the art.

It should be noted that an ultrasound system suitable for use in an implementation of the present invention, and in particular the component structure of the ultrasound systems of FIGURES 2 and 4, may be implemented in hardware, software or a combination thereof. The various embodiments and/or components of an ultrasound system, for example, the fetal bone model and deep learning software modules, or components, processors, and controllers therein, also may be implemented as part of one or more computers or microprocessors. The computer or processor may include a computing device, an input device, a display unit and an interface, for example, for accessing the Internet. The computer or processor may include a microprocessor. The microprocessor may be connected to a communication bus, for example, to access a PACS system or the data network for importing training images. The computer or processor may also include a memory. The memory devices such as the 3D image data memory 32, the training image memory, the clinical data memory, and the memory storing fetal bone model libraries may include Random Access Memory (RAM) and Read Only Memory (ROM). The computer or processor further may include a storage device, which may be a hard disk drive or a removable storage drive such as a floppy disk drive, optical disk drive, solid-state thumb drive, and the like. The storage device may also be other similar means for loading computer programs or other instructions into the computer or processor.

As used herein, the term "computer" or "module" or "processor" or "workstation" may include any processor-based or microprocessor-based system including systems using microcontrollers, reduced instruction set computers (RISC), ASICs, logic circuits, and any other circuit or processor capable of executing the functions described herein. The above examples are exemplary only, and are thus not intended to limit in any way the definition and/or meaning of these terms.

The computer or processor executes a set of instructions that are stored in one or more storage elements, in order to process input data. The storage elements may also store data or other information as desired or needed. The storage element may be in the form of an information source or a physical memory element within a processing machine.

The set of instructions of an ultrasound system including those controlling the acquisition, processing, and transmission of ultrasound images as described above may include various commands that instruct a computer or processor as a processing machine to perform specific operations such as the methods and processes of the various embodiments of the invention. The set of instructions may be in the form of a software program. The software may be in various forms such as system software or application software and which may be embodied as a tangible and non-transitory computer readable medium. Further, the software may be in the form of a collection of separate programs or modules such as a neural network model module, a program module within a larger program or a portion of a program module. The software also may include modular programming in the form of object-oriented programming. The processing of input data by the processing machine may be in response to operator commands, or in response to results of previous processing, or in response to a request made by another processing machine.

## Claims

1. An ultrasonic imaging system which sets or recommends acoustic output levels or output limits in consideration of image data, the system comprising:
an ultrasound probe (10) adapted to acquire image data of an anatomy, wherein the probe comprises a transducer array (112) adapted to transmit acoustic waves of a controllable acoustic output;
a display (100) adapted to display an ultrasound image of the anatomy from the image data;
an image recognition processor (80, 86) adapted to process the image data to recognize a characteristic of the anatomy to identify the anatomy; and
an acoustic output controller (44) adapted to recommend or set an acoustic output level or output limit for the transducer array in consideration of the identified anatomy;
wherein the acoustic output controller (44) is further adapted to cause an acoustic output message to be displayed on the display that the acoustic output may be increased when operating below recommended acoustic output limits.

2. The ultrasonic imaging system of Claim 1, wherein the image recognition processor (80, 86) further comprises an anatomical model (86).

3. The ultrasonic imaging system of Claim 2, wherein the image recognition processor (80, 86) is further adapted to compare the image data with the anatomical model.

4. The ultrasonic imaging system of Claim 3, wherein the image recognition processor (80, 86) is adapted to characterize the ultrasound image in response to the comparison of the image data with an anatomical model.

5. The ultrasonic imaging system of Claim 1, wherein the image recognition processor (80, 86) comprises a neural network model.

6. The ultrasonic imaging system of Claim 5, wherein the image recognition processor (80, 86) further comprises a training image memory (82).

7. The ultrasonic imaging system of Claim 5, wherein the image recognition processor (80, 86) is further adapted to characterize the ultrasound image in response to deep learning analysis of the ultrasound image by the neural network model (80).

8. The ultrasonic imaging system of Claim 1, further comprising a memory (38) in communication with the acoustic output controller (44) which is adapted to store data of clinical acoustic output limits,
wherein the acoustic output controller is further adapted to recommend or set the acoustic output level or output limit for the transducer array in consideration of the data.

9. The ultrasonic imaging system of Claim 1, further comprising a transmit controller (18) coupled to the transducer array (112) and adapted to control acoustic transmission by the transducer array,
wherein the transmit controller is responsive to acoustic output limits set by the acoustic output controller (44).

10. The ultrasonic imaging system of Claim 9, wherein the acoustic output controller (44) is further responsive to one or more of transducer drive voltage, imaging mode, pulse repetition frequency, focus depth, pulse length, and transducer type in recommending or setting an acoustic output level or output limit.

11. The ultrasonic imaging system of Claim 1, wherein the acoustic output controller (44) is further adapted to inhibit an imaging mode in response to a characterization of an image.

12. A computer-implemented method for setting or recommending acoustic output levels or output limits in consideration of image data, the method comprising the steps of:
acquiring (60) image data from an ultrasound probe in an ultrasound system;
processing the image data (62) to recognize a characteristic of an anatomy to identify the anatomy being imaged;
providing, based on the identified anatomy, at least one of: an output guidance to adjust the acoustic output level or automatically adjusting the acoustic output level;
wherein the method further comprises causing an acoustic output message to be displayed on a display that the acoustic output may be increased when operating below recommended acoustic output limits.

13. The method of claim 12, further comprising comparing (64) the characteristic of the acoustic output level to a predetermined clinical limit for the anatomy being imaged; and
providing the output guidance or the adjustment of the acoustic output level based on the comparison.

14. A computer program product embodied in a non-volatile computer readable medium and providing instructions for a processor to carry out the method of claim 12 or 13.

## Patentansprüche

1. Ultraschallbildgebungssystem, das akustische Ausgangspegel oder Ausgangsgrenzwerte unter Berücksichtigung von Bilddaten festlegt oder empfiehlt, wobei das System Folgendes umfasst:
eine Ultraschallsonde (10), die so ausgelegt ist, dass sie Bilddaten einer Anatomie erfasst, wobei die Sonde ein Wandlerarray (112) umfasst, das so ausgelegt ist, dass es Schallwellen mit steuerbarem akustischen Ausgang überträgt;
eine Anzeige (100), das so ausgelegt ist, dass es ein Ultraschallbild der Anatomie aus den Bilddaten anzeigt;
einen Bilderkennungsprozessor (80, 86), der so ausgelegt ist, dass er die Bilddaten verarbeitet, um ein anatomisches Merkmal zu erkennen, um die Anatomie zu identifizieren; und
eine akustische Ausgangssteuereinheit (44), die so ausgelegt ist, dass sie unter Berücksichtigung der ermittelten Anatomie einen akustischen Ausgangspegel oder einen Ausgangsgrenzwert für die Wandleranordnung empfiehlt oder festlegt;
wobei die akustische Ausgangssteuereinheit (44) ferner so ausgelegt ist, dass sie bewirkt, dass auf dem Display eine akustische Ausgangsmeldung angezeigt wird, dass der akustische Ausgangspegel erhöht werden kann, wenn der Betrieb unterhalb der empfohlenen akustischen Ausgangsgrenzwerte liegt.

2. Ultraschallbildgebungssystem nach Anspruch 1, wobei der Bilderkennungsprozessor (80, 86) ferner ein anatomisches Modell (86) umfasst.

3. Ultraschallbildgebungssystem nach Anspruch 2, wobei der Bilderkennungsprozessor (80, 86) ferner so ausgelegt ist, dass er die Bilddaten mit dem anatomischen Modell vergleicht.

4. Ultraschallbildgebungssystem nach Anspruch 3, wobei der Bilderkennungsprozessor (80, 86) so ausgelegt ist, dass er das Ultraschallbild in Reaktion auf den Vergleich der Bilddaten mit einem anatomischen Modell charakterisiert.

5. Ultraschallbildgebungssystem nach Anspruch 1, wobei der Bilderkennungsprozessor (80, 86) ein neuronales Netzwerkmodell umfasst.

6. Ultraschallbildgebungssystem nach Anspruch 5, wobei der Bilderkennungsprozessor (80, 86) ferner einen Trainingsbildspeicher (82) umfasst.

7. Ultraschallbildgebungssystem nach Anspruch 5, wobei der Bilderkennungsprozessor (80, 86) ferner so ausgelegt ist, dass er das Ultraschallbild in Reaktion auf eine Deep-Learning-Analyse des Ultraschallbildes durch das neuronale Netzwerkmodell (80) charakterisiert.

8. Ultraschallbildgebungssystem nach Anspruch 1, ferner umfassend einen Speicher (38), der mit der akustischen Ausgangssteuereinheit (44) in Verbindung steht und zur Speicherung von Daten über klinische akustische Ausgangsgrenzwerte ausgelegt ist,
wobei die akustische Ausgangssteuereinheit ferner so ausgelegt ist, dass sie unter Berücksichtigung der Daten den akustischen Ausgangspegel oder den Ausgangsgrenzwert für das Wandlerarray empfiehlt oder festlegt.

9. Ultraschallbildgebungssystem nach Anspruch 1, ferner umfassend eine Sendesteuereinheit (18), die mit dem Wandlerarray (112) gekoppelt und so ausgelegt ist, dass sie eine akustischen Übertragung durch das Wandlerarray steuert,
wobei die Sendesteuereinheit auf die von der akustischen Ausgangssteuereinheit (44) festgelegten akustischen Ausgangsgrenzwerte reagiert.

10. Ultraschallbildgebungssystem nach Anspruch 9, wobei die akustische Ausgangssteuereinheit (44) ferner auf eine oder mehrere von Wandleransteuerspannung, Bildgebungsmodus, Impulsfolgefrequenz, Fokustiefe, Impulslänge und Wandlertyp beim Empfehlen oder Festlegen eines akustischen Ausgangspegels oder eines Ausgangsgrenzwerts reagiert.

11. Ultraschallbildgebungssystem nach Anspruch 1, wobei die akustische Ausgangssteuereinheit (44) ferner so ausgelegt ist, dass sie einen Bildgebungsmodus in Reaktion auf eine Charakterisierung eines Bildes unterdrückt.

12. Computergestütztes Verfahren zum Festlegen oder Empfehlen von akustischen Ausgangspegeln oder Ausgangsgrenzwerten unter Berücksichtigung von Bilddaten, wobei das Verfahren die folgenden Schritte umfasst:
Erfassen (60) von Bilddaten von einer Ultraschallsonde in einem Ultraschallsystem;
Verarbeiten der Bilddaten (62), um ein anatomisches Merkmal zu erkennen, um die abgebildete Anatomie zu identifizieren;
Bereitstellen, auf Grundlage der identifizierten Anatomie, mindestens einer von: einer Ausgabeanleitung zur Anpassung des akustischen Ausgangspegels oder einer automatischen Anpassung des akustischen Ausgangspegels;
wobei das Verfahren ferner Bewriken umfasst, dass eine akustische Ausgabemeldung auf einer Anzeige angezeigt wird, dass der akustische Ausgang erhöht werden kann, wenn der Betrieb unterhalb der empfohlenen akustischen Ausgangsgrenzwerte liegt.

13. Verfahren nach Anspruch 12, ferner umfassend Vergleichen (64) des Merkmals des akustischen Ausgangspegels mit einem vorbestimmten klinischen Grenzwert für die abgebildete Anatomie; und
Bereitstellen der Ausgangsanleitung oder der Anpassung des akustischen Ausgangspegels auf der Grundlage des Vergleichs.

14. Computerprogrammprodukt, das in einem nichtflüchtigen computerlesbaren Medium verkörpert ist und Anweisungen für einen Prozessor zum Ausführen des Verfahrens nach Anspruch 12 oder 13 bereitstellt.

## Revendications

1. Système d'imagerie ultrasonore qui définit ou recommande des niveaux ou des limites de sortie acoustiques en tenant compte des données d'image, ce système comprenant :
une sonde à ultrasons (10) adaptée à l'acquisition de données d'image d'une anatomie, dans lequel la sonde comprend un réseau de transducteurs (112) adapté à la transmission d'ondes acoustiques d'une sortie acoustique contrôlable ;
un écran (100) adapté pour afficher une image ultrasonore de l'anatomie à partir des données d'image ;
un processeur de reconnaissance d'images (80, 86) adapté au traitement des données d'image pour reconnaître une caractéristique de l'anatomie afin d'identifier l'anatomie ; et
un dispositif de commande de sortie acoustique (44) adapté pour recommander ou définir un niveau de sortie acoustique ou une limite de sortie pour le réseau de transducteurs en tenant compte de l'anatomie identifiée ;
dans lequel le dispositif de commande de sortie acoustique (44) est en outre adapté pour faire en sorte qu'un message de sortie acoustique s'affiche sur l'écran indiquant que la sortie acoustique peut être augmentée en cas de fonctionnement en dessous des limites de sortie acoustique recommandées.

2. Système d'imagerie ultrasonore selon la revendication 1, dans lequel le processeur de reconnaissance d'images (80, 86) comprend en outre un modèle anatomique (86).

3. Système d'imagerie ultrasonore selon la revendication 2, dans lequel le processeur de reconnaissance d'images (80, 86) est en outre adapté pour comparer les données d'image avec le modèle anatomique.

4. Système d'imagerie ultrasonore selon la revendication 3, dans lequel le processeur de reconnaissance d'images (80, 86) est adapté pour caractériser l'image ultrasonore en réponse à la comparaison des données d'image avec un modèle anatomique.

5. Système d'imagerie ultrasonore selon la revendication 1, dans lequel le processeur de reconnaissance d'images (80, 86) comprend un modèle de réseau neuronal.

6. Système d'imagerie ultrasonore selon la revendication 5, dans lequel le processeur de reconnaissance d'images (80, 86) comprend en outre une mémoire d'images d'apprentissage (82).

7. Système d'imagerie ultrasonore selon la revendication 5, dans lequel le processeur de reconnaissance d'images (80, 86) est en outre adapté pour caractériser l'image ultrasonore en réponse à une analyse d'apprentissage profond de l'image ultrasonore par le modèle de réseau neuronal (80).

8. Système d'imagerie ultrasonore selon la revendication 1, comprenant en outre une mémoire (38) en communication avec le dispositif de commande de sortie acoustique (44) qui est adapté pour stocker des données de limites de sortie acoustique clinique,
dans lequel le dispositif de commande de sortie acoustique est en outre adapté pour recommander ou définir le niveau de sortie acoustique ou la limite de sortie du réseau de transducteurs en tenant compte des données.

9. Système d'imagerie ultrasonore selon la revendication 1, comprenant en outre un dispositif de commande d'émission (18) couplé au réseau de transducteurs (112) et adapté pour commander la transmission acoustique par le réseau de transducteurs,
dans lequel le dispositif de commande de transmission est sensible aux limites de sortie acoustique fixées par le dispositif de commande de sortie acoustique (44).

10. Système d'imagerie ultrasonore selon la revendication 9, dans lequel le dispositif de commande de sortie acoustique (44) est en outre sensible à un ou plusieurs parmi une tension d'entraînement du transducteur, un mode d'imagerie, une fréquence de répétition des impulsions, une profondeur de mise au point, une longueur d'impulsion et un type de transducteur, pour recommander ou définir un niveau de sortie acoustique ou une limite de sortie.

11. Système d'imagerie ultrasonore selon la revendication 1, dans lequel le dispositif de commande de sortie acoustique (44) est en outre adapté pour inhiber un mode d'imagerie en réponse à une caractérisation d'une image.

12. Procédé informatisé de réglage ou de recommandation de niveaux ou de limites de sortie acoustiques en tenant compte de données d'image, le procédé comprenant les étapes suivantes :
l'acquisition (60) de données d'image à partir d'une sonde à ultrasons dans un système à ultrasons ;
le traitement des données d'image (62) pour reconnaître une caractéristique d'une anatomie afin d'identifier l'anatomie imagée ;
la fourniture, sur la base de l'anatomie identifiée, d'au moins l'un des éléments suivants : un guide de sortie pour ajuster le niveau de sortie acoustique ou un réglage automatique du niveau de sortie acoustique ;
dans lequel le procédé comprend en outre l'affichage d'un message indiquant que le niveau de sortie acoustique peut être augmenté lorsque le fonctionnement est inférieur aux limites de sortie acoustique recommandées.

13. Procédé selon la revendication 12, comprenant en outre la comparaison (64) de la caractéristique du niveau de sortie acoustique à une limite clinique prédéterminée pour l'anatomie imagée ; et
la fourniture des indications sur le niveau de sortie ou l'ajustement du niveau de sortie acoustique en fonction de la comparaison.

14. Produit de programme informatique incorporé dans un support non volatil lisible par ordinateur et fournissant des instructions à un processeur pour exécuter le procédé selon la revendication 12 ou 13.
